# EUROPEAN PATENT APPLICATION

(11) **EP 2 594 141 A1**
(43) Date of publication of application: **22.05.2013**
(21) Application number: 11189191.7
(22) Date of filing: 15.11.2011
(51) Int. Cl.: A23L 1/03, A61K 31/122, A61K 31/198, A61K 31/225, A61K 31/375, A61K 31/455, A61K 31/522, A61P 39/06

(54) **Composition for protection against cell-damaging effects**

(71) Applicant: TIMA Foundation, 9496 Balzers (LI)
(72) Inventor: Inufusa, Haruhiko, Osaka 542-0081 (JP)
(74) Representative: Dehmel, Albrecht

(57) **Abstract**

The present invention relates to the use of a composition comprising Vitamin C, L-glutamine, L-cystine or L-cysteine, riboflavin, succinic acid, fumaric acid, coenzyme Q10, and niacin for minimizing the cell-damaging effect of radicals and harmful forms of oxygen, in particular of oxygen radicals or reactive oxygen species. The present invention relates further to the composition for use in the treatment and/or prevention of radical and harmful forms of oxygen derived diseases, in particular of oxygen radical or reactive oxygen species derived diseases. The present invention relates further to the composition as a food supplement or dietary supplement.

## Description

The present invention relates to the use of a composition comprising Vitamin C, L-glutamine, L-cystine or L-cysteine, riboflavin, succinic acid, fumaric acid, coenzyme Q10, and niacin for minimizing the cell-damaging effect of radicals and harmful forms of oxygen, in particular of oxygen radicals or reactive oxygen species. The present invention relates further to the composition for use in the treatment and/or prevention of radical and harmful forms of oxygen derived diseases, in particular of oxygen radical or reactive oxygen species derived diseases. The present invention relates further to the composition as a food supplement or dietary supplement.

Though oxygen is absolutely required for aerobic life, it can participate in a variety of toxic reactions involving oxygen free radicals and transition metals that damage many biomolecules. In the past, the oxidative damage of a great number of many low-molecular and macromolecular compounds and fluids of biological and technical interest has been investigated by several approaches. The damaging effects were induced by Reactive Oxygen Species (ROS), obtained by radiolysis of water caused e.g. by ionizing radiation such as X- or gamma-radiation or by metal-catalyzed cleavage of hydrogen peroxide (H₂O₂). Among the physicochemical techniques, in particular spectroscopic studies of many biomolecules revealed, effectively and very fast, numerous changes of the local and global structure of the constituents, together with alterations of their functional ability.

Free radicals are highly reactive owing to the presence of unpaired electron(s). Radicals are short-lived; however they attack, effectively and very fast, all other molecules located close to them. Such so-called primary effects occur in subseconds. However, because of chain propagation processes, radicals may also cause damage far in excess of their original reaction products and far away from their initial place of formation. A cascade of uncontrollable reactions will take place after their attack. Such secondary effects occur in seconds to years. Although H₂O₂ belongs to the ROS group it is no radical, i.e. it is less dangerous, but has more time to react and is responsible for many additional secondary effects.

In the case of ROS-treated or irradiated air-saturated aqueous solutions, the most important noxious species involved in the primary damages of biomolecules are hydroxyl radicals (˙OH), and to a minor extent superoxide anion radicals (O₂·-) and hydrogen peroxide (H₂O₂). Hydroxyl radicals (˙OH) are considered to be responsible for the most deleterious, primary effects on biomolecules and for the damages within the body. Consequently, substances which act as strong ˙OH scavengers are most effective in avoiding noxious effects of ionizing radiation, in particular when present in high concentrations. H₂O₂ is responsible for many secondary effects, since it is stable for some time.

Addition of certain compounds to the samples prior to treatment/irradiation (a.r. (ante radiationem i.e. before irradiation) additives) or afterwards (p.r. (post radiationem i.e. after irradiation) additives) unveiled a great number of possible modifications of the occurring damages. These substances may be used for testing the extent of protection and reparability. Modifications of the effects, however, may also be achieved by changes of other environmental conditions such as the type of ROS production or the wavelength of the radiation applied.

In vivo, ROS effects and oxidative damage are suppressed largely by efficient antioxidant defense mechanisms; these defense systems, however, can only work if sufficient amounts of bioprotectives, e.g. scavengers, antioxidants and antioxienzymes are available. Otherwise serious, frequently irreversible damages of biomolecules may occur.

As a consequence of the scavenging process, scavengers themselves are destroyed or at least transformed to compounds which are incapable of further acting as scavengers. The simultaneous presence of putative scavengers in solution may lead to quite different effects, e.g. synergism, additivity, prevalence of one effective scavenger, weakening of the effect of the prevalent scavenger, no effect, or even effects more detrimental than in the absence of any scavenger. Some substances can act as either anti-oxidants or pro-oxidants (some kind of 'radical reversal'), depending on the specific set conditions, e.g. concentration, presence of oxygen or transition metals. In general, higher concentrations of scavengers amplify the protective effects. In some cases, however, the given conditions may lead to antagonistic effects, thereby reducing positive effects. The presence of the enzyme catalase or peroxidase can decompose H₂O₂, thereby impeding the long-term effects of this agent.

Since the naturally occurring defense system of the body is able to combat only certain, rather moderate amounts of ROS (usually occurring in the body), an excess of ROS can only be met by supply of selected additives, preferably antioxidants. This is necessarily the case when high levels of oxidative stress are reached. Otherwise an imbalance between the production and manifestation of ROS and the ability to detoxify the reactive intermediates or to repair the resulting damage will occur. This imbalance may contribute to the aging process and may lead to many human diseases, including heart disease and cancer.

Thus, there is a need to protect a subject against radicals and harmful forms of oxygen.

This object is solved by the subject matter defined in the claims.

The following figures illustrate the present invention.

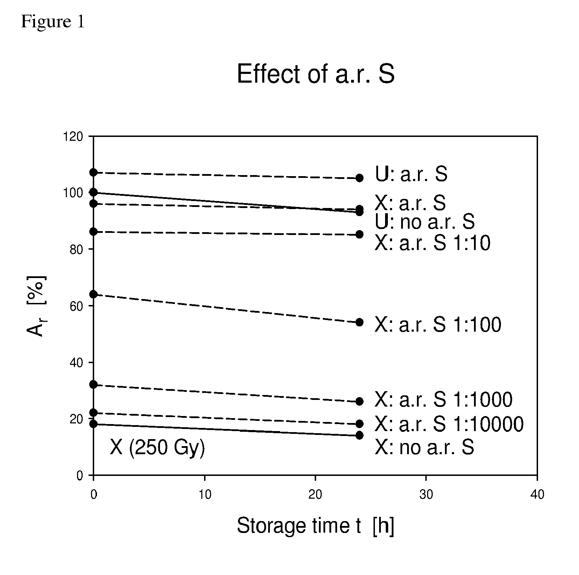

Figure 1 shows in a graphical representation the residual activity (Aᵣ) of unirradiated (U) and X-irradiated lysozyme (c = 0.1 mg/ml) in the post-irradiation phase in the absence or presence of the composition according to the invention before the exposure to radiation. The irradiation of the lysozyme was performed using X-ray doses of 250 Gy. The gray (Gy), with units J/kg, is the SI unit of absorbed dose, which represents the amount of radiation required to deposit 1 joule of energy in 1 kilogram of any kind of matter.

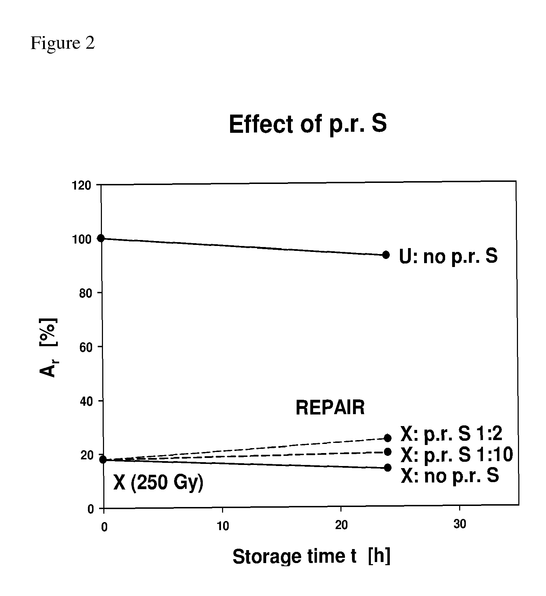

Figure 2 shows in a graphical representation the residual activity (Aᵣ) of unirradiated (U) and X-irradiated lysozyme (c = 0.1 mg/ml) in the post-irradiation phase in the absence or presence of composition according to the invention after the exposure to radiation. The irradiation of the lysozyme was performed using X-ray doses of 250 Gy. The gray (Gy), with units J/kg, is the SI unit of absorbed dose, which represents the amount of radiation required to deposit 1 joule of energy in 1 kilogram of any kind of matter.

The term "composition" as used herein, refers to any kind of composition which could be applied to a subject in any form without causing toxic symptoms in the subject to which physiologically compatible amounts of the respective composition are applied.

The term "supplement", "dietary supplement" or "food supplement" as used herein, refers to a composition which is consumed in addition to the daily meals or in between.

The term "dosage form" as used herein, refers to an amount of medication to be taken at one time, optionally in regular intervals.

The term "minimizing" as used herein, refers to a reduction of the cell-damaging effect caused by radicals in all degrees, i.e. from the total avoiding of the occurrence of cell-damaging effects to the slightest improvement of the cell-damaging effects.

The term "radical" as used herein, refers to atoms, molecules or ions with at least one unpaired electron. Free radicals may have positive, negative, or zero charge. With some exceptions, the unpaired electrons cause radicals to be highly chemically reactive. Radicals containing several unpaired electrons are called diradical or biradical, triradical and so on. Radicals play an important role in specific oxidation processes, in polymerisation and in some substitution reactions. Radicals, if allowed to run free in the body, are believed to be involved in degenerative diseases, senescence (the aging process), and cancers. Radicals may be formed by heat via thermolytic cleavage, electrochemically by oxidation and reduction respectively, and by X-radiation or other ionizing radiation, e.g. α-, β- and γ-radiation.

The term "cell-damaging effects" as used herein, refers to the damage of biomolecules, e.g. nucleic acids, such as DNA and RNA, proteins, enzymes, amino acids, sugars, hormones and metabolites, oxidations of polyunsaturated fatty acids in lipids (lipid peroxidation), oxidations of amino acids in proteins and oxidative inactivation of specific enzymes by oxidation of cofactors.

The term "oxygen radicals" or "reactive oxygen species" as used herein, refers to substances that cause a cell damaging effect e.g. through naturally occurring free radicals or free radicals occurring through heat via thermolytic cleavage, electrochemically through oxidation and reduction respectively, and through X-radiation or other ionizing radiation, e.g. α-, β- and γ-radiation. "Oxygen radicals" or "reactive oxygen species" are both harmful forms of oxygen. Examples include superoxide radicals, hydroxyl radicals, hypochlorite radicals, hydrogen peroxide, various lipid peroxides, peroxy radical, alkoxy radical of lipids, hydroperoxide, ozone and hypochlorite anion, as well as excited oxygen molecules (singlet oxygen).

The term "radical-derived diseases" as used herein refers to a pathological condition of an individual which results at least in part from the production of or exposure to free radicals, particularly oxygen radicals and other reactive oxygen species in vivo. It is evident to those of skill in the art that most pathological conditions are multifactorial, in that multiple factors contributing to the disease state are present. Such diseases encompass pathological states that are recognized in the art as being conditions wherein damage from free radicals, in particular oxygen radicals or reactive oxygen species is believed to contribute to the pathology of the disease state by decreasing symptoms, increasing survival, or providing other detectable clinical benefits in treating or preventing the pathological state.

The present invention relates to a composition comprising Vitamin C, L-glutamine, L-cystine or L-cysteine, riboflavin, succinic acid, fumaric acid, coenzyme Q10, and niacin.

A standard dose comprises about 1.0 g Vitamin C, 1.0 g L-glutamine, 500 mg L-cystine or L-cysteine, 40 mg riboflavin, 100 mg succinic acid or one of its salts (e.g. succinate), 100 mg fumaric acid or one of its salts (e.g. fumarate), 100 mg coenzyme Q10 and 20 mg niacin (Vitamin B3). Preferably the relation of the components of the composition is oriented towards the above given relation. The overall dosage may be adapted to the body mass weight of the consumer.

In a preferred embodiment the standard dose of the composition of the present invention includes 100 mg coenzyme Q10, 40 mg riboflavin, 500 mg L-cystine or L-cysteine, 20 mg niacin, 940 mg Vitamin C, 100 mg succinic acid, 100 mg fumaric acid and 950 mg L-glutamine.

In a preferred embodiment coenzyme Q10 of the composition according to the present invention is provided as CoQ10P40 (Nisshin Pharma, Tokyo, Japan) containing 40% coenzyme Q10.

It is possible to add further substances such as fruit juice extracts, curcuma, tannin, a powder of Panax notoginseng, and Vinca rosea in suitable amounts. Oolong tea, aloe vera and spiral water algae might also be added.

Preferably, the composition according to the present invention comprises a niacin fraction in a quantity ranging from and including 0.05 to 2 mg/kg body weight, 0.1 to 1.5 mg/kg body weight or 0.2 to 0.75 mg/kg body weight, preferably 0.25 mg/kg body weight.

Preferably, the composition according to the present invention comprises a L-cystine or L-cysteine fraction in a quantity ranging from and including 1 to 15 mg/kg body weight, 2.5 to 10 mg/kg body weight or 5 to 8 mg/kg body weight, preferably 6.25 mg/kg body weight.

Preferably, the composition according to the present invention comprises a L-glutamine fraction in a quantity ranging from and including 1 to 20 mg/kg body weight, 5 to 15 mg/kg body weight or 7 to 12 mg/kg body weight, preferably 11.875 mg/kg body weight.

Preferably, the composition according to the present invention comprises a riboflavin fraction in a quantity ranging from and including 0.05 to 2 mg/kg body weight, 0.1 to 1.5 mg/kg body weight or 0.2 to 0.75 mg/kg body weight, preferably 0.5 mg/kg body weight.

Preferably, the composition according to the present invention comprises a succinic acid fraction in a quantity ranging from and including 0.1 to 10 mg/kg body weight, 0.5 to 5 mg/kg body weight or 0.75 to 2 mg/kg body weight, preferably 1.25 mg/kg body weight.

Preferably, the composition according to the present invention comprises a fumaric acid fraction in a quantity ranging from and including 0.1 to 10 mg/kg body weight, 0.5 to 5 mg/kg body weight or 0.75 to 2 mg/kg body weight, preferably 1.25 mg/kg body weight.

Preferably, the composition according to the present invention comprises a coenzyme Q10 fraction in a quantity ranging from and including 0.1 to 10 mg/kg body weight, 0.5 to 5 mg/kg body weight or 0.75 to 2 mg/kg body weight, preferably 1.25 mg/kg body weight.

Preferably, the composition according to the present invention comprises a Vitamin C fraction in a quantity ranging from and including 1 to 20 mg/kg body weight, 5 to 15 mg/kg body weight or 7 to 12 mg/kg body weight, preferably 11.75 mg/kg body weight.

Preferably, the composition is constituted in a manner wherein a dosage of same includes a Vitamin C fraction of about 34.2 mass% i.e. a quantity of Vitamin C in the range from 0.78 to 1.18 g, preferably 1.0 g, more preferably 940 mg within a dose of 2.75 g for a person with a body weight of about 80 kg.

Preferably, the composition is constituted in a manner wherein a dosage of same includes a L-glutamine fraction of about 34.5 mass%, i.e. a quantity of said L-glutamine fraction in the range from 0.78 to 1.18 g, preferably 1.0 g, more preferably 950 mg within a dose of 2.75 g for a person with a body weight of about 80 kg.

Preferably, the composition is constituted in a manner wherein a dosage of same includes a L-cystine or L-cysteine fraction of about 18.2 mass%, i.e. a quantity of said L-cystine or L-cysteine fraction in the range from 460 to 540 mg, preferably 500 mg, within a dose of 2.75 g for a person with a body weight of about 80 kg.

Preferably, the composition is constituted in a manner wherein a dosage of same includes a riboflavin fraction of about 1.45 mass% i.e. a quantity of said riboflavin in the range from 32 to 48 mg, preferably 40mg, within a dose of 2.75 g for a person with a body weight of about 80 kg.

Preferably, the composition is constituted in a manner wherein a dosage of same includes a succinic acid fraction or one of its salts (e.g. succinate) of about 3.64 mass%, i.e. a quantity of said succinic acid in the range from 90 to 110 mg, preferably 100 mg, within a dose of 2.75 g for a person with a body weight of about 80 kg.

Preferably, the composition is constituted in a manner wherein a dosage of same includes a fumaric acid fraction or one of its salts (e.g. fumarate) of about 3.64 mass%, i.e. a quantity of said fumaric acid in the range from 90 to 110 mg, preferably 100 mg, within a dose of 2.75 g for a person with a body weight of about 80 kg.

Preferably, the composition is constituted in a manner wherein a dosage of same includes a coenzyme Q10 fraction of about 3.64 mass%, i.e. a quantity of said coenzyme fraction in the range from 30 to 300 mg, preferably 250 mg, within a dose of 2.75 g for a person with a body weight of about 80 kg.

Preferably, the composition is constituted in a manner wherein a dosage of same includes a niacin fraction of about 0.73 mass%, i.e. a quantity of said coenzyme fraction in the range from 1 to 40 mg, preferably 20 mg, within a dose of 2.75 g for a person with a body weight of about 80 kg.

More preferably each ingredient of the composition is in the range from about 0.01 to about 100 gram, preferably from about 0.05 to 50 gram.

In a preferred embodiment the composition according to the invention is provided in liquid form. The composition according to the invention in liquid form may additionally comprise a buffer system for solubility reasons. Preferably the buffer system is bicarbonate, phosphate or any other buffering system. In another preferred embodiment the composition according to the invention is provided in liquid form with added bicarbonate, preferably the standard dose of the composition according to the invention comprises a sodium bicarbonate fraction ranging from and including 0.2 to 10 mg/kg body weight, 0.5 to 5 mg/kg body weight or 1 to 4 mg/kg body weight, preferably 0.95 mg/kg body weight. In another preferred embodiment the liquid composition according to the invention is constituted in a manner wherein a dosage of same includes a sodium bicarbonate fraction of about 2.96 mass%, i.e. a quantity of said sodium bicarbonate fraction in the range from 1 to 100 mg, preferably 84 mg, within a dose of 2.834 g for a person with a body weight of about 80 kg.

In a preferred embodiment the composition according to the invention provided in liquid form comprises Vitamin C, L-glutamine, L-cystine, riboflavin, succinic acid, fumaric acid, coenzyme Q10, and niacin. More preferably, the composition according to the invention provided in liquid form comprises Vitamin C, L-glutamine, L-cystine, riboflavin, succinic acid, fumaric acid, coenzyme Q10, niacin and a buffer system for solubility reasons. Preferably the buffer system is bicarbonate, phosphate or any other buffering system.

It is possible to add further substances such as fruit juice extracts, curcuma, tannin, a powder of Panax notoginseng, and Vinca rosea in suitable amounts. Oolong tea, aloe vera and spiral water algae might also be added.

The composition according to the present invention may be administered by any route of administration, preferably orally.

The composition according to the present invention may be provided in form of a tablet, capsule, dragee, pill, powder, suppository, or any other galenic formulation. The tablet, capsule, dragee, pill, powder, suppository, or any other galenic formulation may be contained in a dosage receptacle. Preferably, the dosage form may comprise suitable carriers, stabilizers, flavourings, buffers or other suitable reagents. The standard dosage of the composition of the invention may be provided in one or more tablets, capsules, dragees, pills, powders, suppositories, or any other galenic formulations, preferably in 5 tablets, capsules, dragees, pills, powders, suppositories, or any other galenic formulations.

The composition according to the present invention may be administered or taken prior, during or after occurrence of radicals or species of the reactive radical group, preferably oxygen radicals or reactive oxygen species or other harmful forms of oxygen. The composition according to the present invention may be taken or administered to any subject, preferably to mammals, more preferably to humans.

Another aspect of the present invention refers to the use of the composition according to the invention for minimizing the cell-damaging effects caused by radicals and harmful forms of oxygen. In a preferred embodiment the composition according to the present invention is used for minimizing the cell-damaging effects caused by harmful forms of oxygen, in particular of oxygen radicals or reactive oxygen species, more preferably by superoxide radicals, hydroxyl radicals, hypochlorite radicals, hydrogen peroxide, various lipid peroxides, peroxy radical, alkoxy radical of lipids, hydroperoxide, ozone and hypochlorite anion, as well as excited oxygen molecules (singlet oxygen). Such radicals and substances may be formed inter alia by radiation, preferably by X-radiation or other ionizing radiation, e.g. α-, β- and γ-radiation, more preferably the radiation may exhibit a wave length of 10 nm to 10 pm.

In a preferred embodiment the composition according to the present invention is used within the context of a radiation therapy for tumour patients.

In a preferred embodiment the composition according to the present invention is used within the context of a chemotherapy for tumour patients.

The composition according to the present invention may be administered or taken prior, during or after a radiation therapy or chemotherapy for tumour patients.

In a further embodiment the composition of the present invention can also be used as a medicament, food supplement and/or dietary supplement.

A further embodiment refers to the composition of the present invention for use in the treatment and/or prevention of radical or harmful forms of oxygen derived diseases in general. A preferred embodiment refers to the composition of the present invention for use in the treatment and/or prevention of oxygen radical or reactive oxygen species derived diseases. A further embodiment refers to the composition of the present invention for use in the treatment and/or prevention of ischemic reperfusion injury, inflammatory diseases, systemic lupus erythematosis, myocardial infarction, stroke, traumatic hemorrhage, spinal cord trauma, Crohn's disease, autoimmune diseases, e.g. rheumatoid arthritis and diabetes, cataract formation, uveitis, emphysema, gastric ulcers, oxygen toxicity, neoplasia, radiation sickness, cancer, Alzheimer's disease, Parkinson's disease, hemochromatosis, deafness, atherosclerosis, psychosis, movement disorders and schizophrenia.

Excessive amounts of free radicals, in particular of oxygen radicals or reactive oxygen species can lead to cell injury and death, which results in many diseases such as stroke, cancer, diabetes and major disorders. Many forms of cancer are thought to be the result of reactions between free radicals, in particular of oxygen radicals or reactive oxygen species and DNA, resulting in mutations that can adversely affect the cell cycle and potentially lead to malignancy.

The amount of free radicals, in particular oxygen radicals or reactive oxygen species may be enhanced in subjects during diagnostic radiology or in case of radiation therapy in connection with tumor diseases. Consequently, radiologists, dentists, radiological assistances and workers in plants for X-ray tubes and nuclear power may exhibit higher amounts of free radicals, in particular of oxygen radicals or reactive oxygen species. Moreover, also personnel in airplanes and spacecrafts are exposed to radiation and thus, may exhibit higher amounts of free radicals, in particular of oxygen radicals or reactive oxygen species. And the same holds true for mountaineers. There is also overwhelming evidence that there is a causative role of smoking-generated oxygen free radicals (huge oxidative stress load) in the pathogenesis of many chronic degenerative diseases, e.g. emphysema, lung cancer, coronary artery diseases.

Radicals produced e.g. by radiolysis of water by radiation, e.g. X-rays, α-, β- and γ-radiation damages e.g. DNA causing mutation of the DNA which may lead to the development of divers diseases, e.g. tumors. Moreover, radicals cause or promote damages, such as degeneration, atrophy, fibrosis or necrosis of tissues.

In a further embodiment, the composition of the present invention is used in the prevention of aging. Organisms age because cells accumulate free radical, in particular oxygen radical or reactive oxygen species damage over time. Free radical, in particular oxygen radical or reactive oxygen species damage within cells has been linked to a range of disorders including cancer, arthritis, atherosclerosis, Alzheimer's disease, and diabetes. Free radical chemistry is an important aspect of phagocytosis, inflammation, and apoptosis. Cell suicide, or apoptosis, is the body's way of controlling cell death and involves free radicals, in particular oxygen radical or reactive oxygen species. Such degenerative diseases associated with aging may be caused by radicals, in particular by oxygen radicals or reactive oxygen species.

A further embodiment refers to the composition according to the present invention for use as a food supplement or dietary supplement. Food supplements and dietary supplements may be concentrated sources of nutrients or other substances with a nutritional or physiological effect whose purpose is to supplement the normal diet.

The food supplement or dietary supplement taken orally may supplement the diet. The dietary supplement or food supplement may be provided as a tablet, capsule, dragee, pill, powder, suppository, or any other galenic formulation. Dietary supplements and food supplements may also be provided in bars, drinks, shakes and other food products. In general, a dietary supplement is not intended to be the sole item of a meal or diet. Dietary supplements and food supplements may be taken before, after or simultaneously with a meal. Dietary supplements and food supplements may be taken once or several times a day. Dietary supplements and food supplements may be taken at any time of day.

The following examples explain the present invention but are not considered to be limiting.

Primary goal of this study was the investigation of changes in X-irradiated biomolecules in the absence and presence of the composition according to the present invention and various other additives, to address the following problems: Alleged protective efficiency of the additives during and after irradiation (elucidation of primary and secondary damages) and their repair capability.

### Example 1: Preparation of the supplement solution

The supplement consists of the following ingredients:

| | |
|---|---|
| Coenzyme Q10* | 100 mg (CoQ10P40 250 mg) |
| Riboflavin | 40 mg (riboflavin 40 mg equivalent) |
| L-Cystine | 500 mg |
| Niacin | 20 mg |
| Ascorbic acid | 940 mg |
| Succinic acid | 100 mg |
| Fumaric acid | 100 mg |
| L-Glutamine | 950 mg |

Ingredients of the supplement except Coenzyme Q10 were purchased from Sigma Aldrich Japan (Tokyo, Japan). *CoQ10P40 (Nisshin Pharma, Tokyo Japan) was used as Coenzyme Q 10. CoQ10P40 contains 40% weight of Coenzyme Q10.

### Example 2: Activity test of lysozyme

The activity test of lysozyme was taken as efficient test system.

The enzyme lysozyme was chosen, because lysozyme is a well-characterized protein, both from the biochemical, physico-chemical, crystallographic and radiobiological point of view. Moreover, lysozyme is an example of a self-defence system against bacterial infections in the body. Structure and function of unirradiated and irradiated lysozyme have been characterized in detail. As follows from previously published papers any other protein could be used as well, with gradual differences caused by the characteristics (e.g., amino acid composition, 3D structure) of the protein selected. This behavior can be attributed to the fact that the principles of the interaction of ionizing radiation and biomolecules are governed by the oxidizing action of ROS, on the one hand, and the scavenging ability of antioxidants, on the other, irrespective of the biomolecule(s) in its neighbourhood. If a scavenger is able to protect a protein, it will also be able to protect any other molecules, e.g. DNA, lipid, membrane, etc., depending on the extent of solubility of the antioxidant applied.

The hen's egg white lysozyme was purchased from Serva (Heidelberg, Germany), additives and all other substances were of the highest available grade. Lysozyme (0.1 mg/ml) and the additives (10 mM, except otherwise stated) were dissolved in dilute, air-saturated 1 mM sodium phosphate buffer pH 6.5 and were irradiated at 25°C with X-rays (0-1 kGy) in the absence or presence of the additives. Activity tests of the lysozyme were performed as described in Shugar, 1952. The measurement of lysozyme activity and the ultra-violet inactivation of lysozyme. Biochim. Biophys. Acta 8, 302-309. were recorded in a Jasco UV/VIS spectrophotometer V-530.

The activity test of lysozyme is simple and straightforward. The method described by Shugar uses the diluted unirradiated or irradiated enzyme and a cell suspension of Micrococcus lysodeikticus in a special activity buffer (66 mM sodium phosphate buffer with 17 mM NaCl, pH 7.0). The bacterium is suspended in an Erlenmeyer flask in the activity buffer at a concentration of 0.2 mg/ml. Before using the bacterial suspension in the test, the flask has to be swivelled to ensure homogeneous pipetting into the cuvette. Working briskly, the enzyme sample was pipetted to the bacterial solution, and using parafilm to tightly seal the cuvette, was homogeneously hand-mixed. Typical test mixtures (total of 3 ml) consisted of 2.98 ml bacterial solution plus 0.02 ml unirradiated enzyme sample in the absence or presence of additives, and 2.95 ml bacterial solution plus 0.05 ml irradiated enzyme (250 Gy) in the absence of additives, and 2.6 ml bacterial solution plus 0.4 ml irradiated enzyme (1000 Gy) in the absence of additives, to allow reasonable handling of the samples. The decrease in absorbance resulting from a breakdown of the bacteria by the enzyme is measured in a 3-ml cuvette in a spectrophotometer at a wavelength of 450 nm for a continuous time period of 3 min at 25°C.

Different decreases in absorbance reflect different activities. The test may also be applied for lysozyme in the presence of antioxidants, since, owing to the high dilution of the samples (lysozyme + antioxidants) in the test and the wavelength used, the antioxidants will not disturb the results of the activity measurement. No further separation techniques are needed.

Irradiations were performed in small 2-ml plastic cups at 25°C. The dosage measurement of X-rays was obtained by Fricke dosimetry (ferrous sulfate dosimetry).

### Example 3: Activity of lysozyme irradiated after application of tested additives

In the present experiment the lysozyme was mixed with the following additives: composition of example 1, ascorbic acid (10 mM), glutathione (10 mM), thiourea (10 mM), sodium-formate (10 mM) or mannitol (10 mM). Afterwards the lysozyme-additive mixture was irradiated with X-rays of an energy dose of 500 Gy and 1000 Gy, respectively. Additionally, the lysozyme was irradiated with X-rays of energy doses of 250, 500, 750 and 1000 Gy without the presence of any additives.

Afterwards the irradiated lysozyme was used in the activity assay as described in example 2 to determine the activity of the lysozyme. For comparison, activities are given in percent. The native, unirradiated lysozyme is used as reference and is considered to have 100 % activity.

Table 1 shows the results of experiments with irradiated lysozyme (c = 0.1 mg/ml) in the absence or presence of different additives added before irradiation and at different X-ray doses.

X-irradiation in the absence of additives results in a nearly complete loss of activity, if the dose applied is sufficient high (1000 Gy). Irradiation at lower doses yields less pronounced damages. The presence of the composition as described in example 1 exhibits a strong protection against X-irradiation. At lower doses applied, the protection is more pronounced.

The decrease in the activity of lysozyme correlates with the occurrence of radicals produced by radiolysis due to the irradiation. The application of the composition as described in example 1 acts as a scavenger and thus, leads to protection of the lysozyme indicated by high activity of the lysoszyme.

**Table 1: Influence of additives on the activity of irradiated lysozyme**

| **Irradiation** | **a.r. Additive (final conc.)** | **% Activity** |
|---|---|---|
| | | |

| **None** | **None** | **100 (reference)** |
|---|---|---|
| | | |
| X (250 Gy) | None | 13 |
| X (500 Gy) | None | 1.5 |
| X (500 Gy) | S 1:7 dilution (= 1 mg/ml) | 89 |
| X (750 Gy) | None | 0.3 |
| X (1000 Gy) | None | 0.1 |
| X (1000 Gy) | S 1:7 dilution (= 1 mg/ml) | 73 |
| X (1000 Gy) | Ascorbic acid (10 mM) | 90 |
| X (1000 Gy) | Glutathione (10 mM) | 58 |
| X (1000 Gy) | Thiourea (10 mM) | 90 |
| X (1000 Gy) | Sodium-Formate (10 mM) | 75 |
| X (1000 Gy) | Mannitol (10 mM) | 51 |

| | | |
|---|---|---|
| S = composition as described in example 1, a.r. = ante radiationem = before irradiation, gray (Gy) represents the amount of radiation required to deposit 1 joule of energy in 1 kilogram of any kind of matter. | | |

### Example 4: Activity of lysozyme irradiated after application of the composition in variable concentrations

In the present experiment the lysozyme was mixed with the composition of example 1 of various concentrations. Afterwards the lysozyme-additive mixture was irradiated with X-rays of an energy dose of 250 Gy. Additionally, the lysozyme was irradiated with X-rays of an energy dose of 250 Gy without the presence of any additives.

Afterwards the irradiated lysozyme was used in the activity assay as described in example 2 to determine the activity of the lysozyme, wherein the determination of the lysozyme activity was repeated 24 h after irradiation. For comparison, activities are given in percent. The native, unirradiated lysozyme is used as reference and is considered to have 100 % activity.

Table 2 summarizes the results for X-irradiated lysozyme (c = 0.1 mg/ml) using variable concentrations of the composition described in example 1. For this purpose, a dose of 250 Gy was applied, to allow the registration of more accurate results than at 1000 Gy. In the absence of the composition, an inactivation takes place, which is further intensified in the post-irradiation phase (monitoring 24 h after irradiation).

All concentrations of the composition (even a dilution of 1:10000) deliver a protection of the lysozyme during irradiation and against further inactivation during storage for 24 h. The most pronounced effects are obtained when using the highest composition concentrations.

The decrease in the activity of lysozyme correlates with the occurrence of radicals produced by radiolysis due to the irradiation. The application of the composition as described in example 1 acts as a scavenger and thus, leads to protection of the lysozyme indicated by high activity of the lysoszyme.

**Table 2: Influence of the composition on the activity of irradiated lysozyme**

| **Irradiation** | **a.r. Additive (final conc.)** | **% Activity** | **% Activity (24 h)** |
|---|---|---|---|
| | | | |

| **None** | **None** | **100 (reference)** | **93 (slight inactivation)** |
|---|---|---|---|
| **X (250 Gy)** | **None** | **18 (strong inactivation)** | **14 (further inactivation)** |
| | | | |
| None | S undiluted (= 7 mg/ml) | 107 (slight repair) | 105 |
| X (250 Gy) | S undiluted (= 7 mg/ml) | 96 | 94 |
| X (250 Gy) | S 1:10 dilution (= 0.7 mg/ml) | 86 | 85 |
| X (250 Gy) | S 1:100 dilution (= 0.07 mg/ml) | 64 | 54 (further inactivation) |
| X (250 Gy) | S 1:1000 dilution (= 0.007 mg/ml) | 32 | 26 (further inactivation) |
| X (250 Gy) | S 1:10000 dilution (= 0.0007 mg/ml) | 22 | 18 (further inactivation) |

| | | | |
|---|---|---|---|
| S = composition as described in example 1, a.r. = ante radiationem = before irradiation, gray (Gy) represents the amount of radiation required to deposit 1 joule of energy in 1 kilogram of any kind of matter. | | | |

### Example 5: Activity of lysozyme irradiated prior to application of the composition in variable concentrations

In the present experiment the lysozyme was first irradiated with X-rays of an energy dose of 250 Gy. Afterwards the lysozyme was mixed with the composition of example 1 in a 1:2 or 1:10 dilution. Additionally, the lysozyme was irradiated with X-rays of an energy dose of 250 Gy without the presence of any additives.

Afterwards the irradiated lysozyme was used in the activity assay as described in example 2 to determine the activity of the lysozyme, wherein the determination of the lysozyme activity was repeated 24 h after irradiation. For comparison, activities are given in percent. The native, unirradiated lysozyme is used as reference and is considered to have 100 % activity.

Table 3 presents the results for lysozyme (c = 0.1 mg/ml) X-irradiated in the absence of the composition of example 1, but furnished with this composition after irradiation. Activity measurements were performed 24 h after irradiation and addition of the composition. The results prove a protection by the composition against inactivation during storage (24 h) and a significant repair behavior.

The decrease in the activity of lysozyme correlates with the occurrence of radicals produced by radiolysis due to the irradiation. The application of the composition as described in example 1 acts as a scavenger and leads to a repair of the lysozyme indicated by high activity of the lysoszyme.

**Table 3: Repair of the lysozyme activity by application of the compositon**

| **Irradiation** | **p.r. Additive (final conc.)** | **% Activity** | **% Activity (24 h at RT)** |
|---|---|---|---|
| | | | |

| **None** | **None** | **100 (reference)** | **93 (slight inactivation)** |
|---|---|---|---|
| **X (250 Gy)** | **None** | **18 (strong inactivation)** | **14 (further inactivation)** |
| | | | |
| | | | |
| X (250 Gy) | S 1:2 dilution (= 3.5 mg/ml) | | 25 (repair) |
| X (250 Gy) | S 1:10 dilution (= 0.7 mg/ml) | | 20 (repair) |

| | | | |
|---|---|---|---|
| S = composition described in example 1, p.r. = post radiationem = after irradiation, gray (Gy) represents the amount of radiation required to deposit 1 joule of energy in 1 kilogram of any kind of matter. | | | |

## Claims

1. A use of the composition comprising Vitamin C, L-glutamine, L-cystine or L-cysteine, riboflavin, succinic acid, fumaric acid, coenzyme Q10, and niacin for minimizing the cell-damaging effect of radicals and harmful forms of oxygen, in particular of oxygen radicals or reactive oxygen species.

2. The use according to claim 1, wherein the composition comprising Vitamin C, L-glutamine, L-cystine, riboflavin, succinic acid, fumaric acid, coenzyme Q10, and niacin is provided in a liquid form.

3. The use according to claim 1 or 2, wherein the composition is in the form of a capsule, dragee, pill, powder, suppository, or any other galenic formulation.

4. The use according to claim 1 to 3 within the context of a radiation therapy for tumour patients.

5. The use according to claim 1 to 3 within the context of a chemotherapy for tumour patients.

6. The use according to claim 1 to 5 as a food supplement or dietary supplement.

7. A composition comprising Vitamin C, L-glutamine, L-cystine or L-cysteine, riboflavin, succinic acid, fumaric acid, coenzyme Q10, and niacin.

8. The composition according to claim 7 for use as a medicament, food supplement or dietary supplement.

9. The composition according to claim 7 for use in the treatment and/or prevention of radical derived diseases.

10. The composition according to claim 7 for use in the treatment and/or prevention of radicals and harmful forms of oxygen, in particular oxygen radical or reactive oxygen species derived diseases.

11. The composition according to claim 7 for use in the treatment and/or prevention of ischemic reperfusion injury, inflammatory diseases, systemic lupus erythematosis, myocardial infarction, stroke, traumatic hemorrhage, spinal cord trauma, Crohn's disease, autoimmune diseases, e.g. rheumatoid arthritis and diabetes, cataract formation, uveitis, emphysema, gastric ulcers, oxygen toxicity, neoplasia, radiation sickness, cancer, Alzheimer's disease, Parkinson's disease, hemochromatosis, deafness, atherosclerosis, psychosis, movement disorders and schizophrenia.
